# EUROPEAN PATENT APPLICATION

(11) **EP 4 434 463 A1**
(43) Date of publication of application: **25.09.2024**
(21) Application number: 23162874.4
(22) Date of filing: 20.03.2023
(51) Int. Cl.: A61B 6/00, A61B 6/03, H05G 1/54

(54) **X-RAY IMAGING SYSTEM FAILURE DETECTION APPARATUS**

(71) Applicant: Koninklijke Philips N.V., 5656 AG Eindhoven (NL)
(72) Inventor: LIPS, Oliver, Eindhoven (NL); VOGTMEIER, Gereon, Eindhoven (NL); DOUGLAS, Alexander Ulrich, 5656AG Eindhoven (NL); LEUSSLER, Christoph Günther, Eindhoven (NL); FORTHMANN, Peter, Eindhoven (NL); RIBBING, Carolina Maria, Eindhoven (NL); WEISS, Steffen, Eindhoven (NL); SCHIENAGEL, Ina, EIndhoven (NL); KAHL, Christoph, Eindhoven (NL)
(74) Representative: Philips Intellectual Property & Standards

(57) **Abstract**

The invention relates to an X-ray imaging system failure detection apparatus. A transceiver system is configured to be located relative to a component (50) of the X-ray imaging system (60). The transceiver system is configured to emit RF radiation. The transceiver system is configured to irradiate at least one part of the component with first RF radiation that is at least a portion of the emitted RF radiation. The transceiver system is configured to collect second RF radiation, wherein the second RF radiation is at least a portion of the first RF radiation that is reflected and/or transmitted by the component. The transceiver system is configured to provide the analyser with data indicative of the second RF radiation. An analyser is configured to determine that the component has a fault or is developing a fault using the data indicative of the second RF radiation. The output unit is configured to output information that the component has a fault or is developing a fault upon the determination by the analyser that the component has a fault or is developing a fault.

## Description

### FIELD OF THE INVENTION

The present invention relates to an X-ray imaging system failure detection apparatus, a medical imaging system, an X-ray imaging system failure detection method, a computer program element, and a computer readable medium.

### BACKGROUND OF THE INVENTION

X-ray imaging systems are subject to multiple degradation modes and failures. In the medical field, such X-ray imaging systems use X-rays to perform medical imaging examinations. X-rays are generated in an evacuated tube. A heated filament is disposed within the tube and is located in a high electric field. Free electrons exiting the filament are accelerated by the electric field towards an anode, where they are stopped and emit bremsstrahlung X-ray radiation that is then used for imaging. A common type of tube is a glass tube that is sometimes surrounded by a metal chassis. A space between the glass tube and the chassis is sometimes filled with a cooling medium such as oil. Imperfections, and also aging of the tube can give rise to arcing, an effect that is due to residual particles that become ionized by the electric field. Arcing disrupts the X-ray generation process. This is detrimental to the imaging examination procedure, which typically relies on a continuous supply of X-rays. Another source of arcing relates to the voltage generator that generates power to the X-ray tube. It has been recognized that air bubbles in the insulating oil of the voltage generator, and also defects in a solid isolating material associated with the voltage generator, can also be a source of arcing. Another cause of arcing is resonance effects of the so-called High Voltage "HV" chain that supplies power to the X-ray tube. High Q (quality factor) resonances of a transformer in the HV chain can be excited by the harmonics of the driving current, resulting in local arcing over windings, or over nearby conductors with low isolation. Arcing can also be caused by general wear and tear, and by soot deposits on connectors associated with the high voltage chain. Furthermore, bearing and filament issues can lead to failure and a disruption of X-ray generation. Similar failure issues that disrupt the X-ray imaging examination occur in other imaging modalities, such as Magnetic Resonance Imaging, "MRI", and Positron Emission Tomography, "PET", imaging.

US 10912181B2 describes a method for detecting high-voltage flashovers in X-ray equipment including an X-ray emitter and a high-voltage supply. It is described that the X-ray emitter has an X-ray tube, surrounded by an insulating medium; and the high-voltage supply has a high-voltage generator and a cable. It is described that the cable is at least part of a connecting passage between the high-voltage generator and the X-ray tube. It is described that during normal operation of the X-ray equipment, an interference pulse, which occurs due to the high-voltage flashover in the connecting passage, is detected and evaluated with the aid of a measuring device, including a measuring element, and an assessment of the condition of the X-ray emitter and of other high voltage-carrying components, and measures that follow, are made using the evaluated interference pulse.

US20220011360A1 describes an arcing detection device. It is described that the arcing detection device may include a detection coil and a processing circuit operably connected to the detection coil. It is described that the detection coil may be configured to detect a current variation of a system. It is described that the processing circuit may be configured to determine information of an arcing event of the system based on the current variation of the system, and that the information of the arcing event of the system may include a position where the arcing event occurs in the system.

US8076943B2 relates to an impedance-based arc detector for CT scanners and method of use and diagnosis therewith, and more specifically, to a two- or three-way conductive probe detector system and associated signal processing unit to distinguish the location of arc faults on a CT scanner at either the high-voltage cable of an x-ray tube, an anode connected to the x-ray tube, a cathode also connected to the x-ray tube, a high-voltage well, or a power distribution unit of the tube.

The degradation and failures of X-ray imaging systems causes downtime and repairs can be cumbersome. If the source of the failure and the component that is failing are not known, an undetected failure can cause further failure and damage

There is a need to address these problems.

### SUMMARY OF THE INVENTION

It would be advantageous to have an improved technique to mitigate the effects of degradation modes and failures in X-ray imaging systems. The object of the present invention is solved with the subject matter of the independent claims, wherein further embodiments are incorporated in the dependent claims.

In a first aspect, there is provided an X-ray imaging system failure detection apparatus, the apparatus comprising:
- a transceiver system;
- an analyser; and
- an output unit.

The transceiver system is configured to be located relative to a component of the X-ray imaging system. The transceiver system is configured to emit RF radiation. The transceiver system is configured to irradiate at least one part of the component with first RF radiation that is at least a portion of the emitted RF radiation. The transceiver system is configured to collect second RF radiation. The second RF radiation is at least a portion of the first RF radiation that is reflected and/or transmitted by the component. The transceiver system is configured to provide the analyser with data indicative of the second RF radiation. The analyser is configured to determine that the component has a fault or is developing a fault. The determination that the component has a fault or is developing a fault comprises utilization of the data indicative of the second RF radiation. The output unit is configured to output information that the component has a fault or is developing a fault upon the determination by the analyser that the component has a fault or is developing a fault.

In an example, the transceiver system is configured to provide the analyser with data indicative of the first RF radiation. The determination that the component has a fault or is developing a fault can then comprise utilization of the data indicative of the first RF radiation to determine a normalized reflectance and/or transmittance.

In an example, the determination that the component has a fault or is developing a fault comprises a utilization of a change in the second RF radiation with respect to reference data.

In an example, the RF radiation comprises radiation emitted over a period of time. The first RF radiation comprises radiation that irradiates the at least one part of the component over the period of time, and the second RF radiation comprises radiation collected over the period of time. The determination that the component has a fault or is developing a fault can then comprise a detection of a change in the second RF radiation over the period of time.

In an example, the detection of the change in the second RF radiation over the period of time comprises a detection of a change in a magnitude and/or phase of the second RF radiation at one or more frequencies.

In an example, the one or more frequencies are resonant frequencies.

In an example, the detection of the change in the second RF radiation over the period of time comprises a detection of a change in a frequency spectrum of the RF radiation.

In an example, the determination that the component has a fault or is developing a fault comprises utilization of data indicative of one or more reference second RF radiation reflected and/or transmitted from the component or a reference component as a consequence of one or more reference first RF radiation having irradiated at least a part of the component or reference component.

In an example, the determination that the component has a fault or is developing a fault comprises utilization of data indicative of the one or more reference first RF radiation.

In an example, the determination that the component has a fault or is developing a fault comprises an identification of the fault or identification of the fault that is developing.

In an example, the analyser is configured to determine that the component has a fault or is developing a fault during operation of the X-ray imaging system; and/or wherein the fault comprises at least one of arcing in an X-ray tube of the X-ray imaging system, arcing in a generator of the X-ray imaging system, degradation of an anode bearing of an X-ray tube of the X-ray imaging system, and degradation of a filament of an X-ray tube of the X-ray imaging system.

In a second aspect, there is provided a medical imaging system, the system comprising:
- an X-ray imaging system;
- a processing unit;
- a transceiver system;
- an analyser; and
- an output unit.

The processing unit is configured to control at least one mode of operation of the X-ray imaging system. The transceiver system is located relative to a component of the X-ray imaging system. The transceiver system is configured to emit RF radiation. The transceiver system is configured to irradiate at least one part of the component with first RF radiation that is at least a portion of the emitted RF radiation. The transceiver system is configured to collect second RF radiation. The second RF radiation is at least a portion of the first HR RF radiation that is reflected and/or transmitted by the component. The transceiver system is configured to provide the analyser with data indicative of the second RF radiation. The analyser is configured to determine that the component has a fault or is developing a fault. The determination that the component has a fault or is developing a fault comprises utilization of the data indicative of the second RF radiation. The output unit is configured to output information that the component has a fault or is developing a fault upon the determination by the analyser that the component has a fault or is developing a fault.

In an example, the processing unit is configured to stop or modify one or more of the at least one mode of operation of the X-ray imaging system comprising utilization of the information that the component has a fault or is developing a fault.

In a third aspect, there is provided an X-ray imaging system failure detection method, the method comprising:
locating a transceiver system relative to a component of the X-ray imaging system;
emitting by the transceiver system RF radiation;
irradiating by the transceiver system at least one part of the component with first RF radiation that is at least a portion of the emitted RF radiation;
collecting by the transceiver system second RF radiation, wherein the second RF radiation is at least a portion of the first RF radiation that is reflected and/or transmitted by the component;
providing by the transceiver system an analyser with data indicative of the second RF radiation;
determining by the analyser that the component has a fault or is developing a fault, wherein the determining that the component has a fault or is developing a fault comprises utilizing the data indicative of the second RF radiation; and
outputting by an output unit information that the component has a fault or is developing a fault upon the determination by the analyser that the component has a fault or is developing a fault.

In an aspect, there is provided a computer program element for controlling an apparatus according to the first aspect which when executed by a processor is configured to carry out the method of the third aspect.

In an aspect, there is provided a computer program element for controlling a system according to the second aspect which when executed by a processor is configured to carry out the method of the third aspect.

Thus, according to aspects, there is provided computer program elements controlling one or more of the apparatuses/systems as previously described which, if the computer program element is executed by a processor, is adapted to perform the method as previously described.

According to another aspect, there is provided computer readable media having stored the computer elements as previously described.

The computer program element can for example be a software program but can also be a Field Programmable Gate Array, "FPGA", a Programmable Logic Device, "PLD", or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Examples of embodiments will be described in the following with reference to the following drawing:
Fig. 1 shows an example of an X-ray imaging system failure detection apparatus;
Fig. 2 shows an example of a medical imaging system;
Fig. 3 shows an example of a medical imaging system;
Fig. 4 shows an example of an X-ray imaging system failure detection method;
Fig. 5 shows a transceiver system located relative to a component of an X-ray imaging system;
Fig. 6 shows examples of RF reflection and transmission spectra versus frequency (MHz) collected at components of an X-ray imaging system in the form of an anode and cathode of an X-ray tube;
Fig. 7 shows examples of RF reflection spectra versus frequency (MHz) collected at a component of an X-ray imaging system in the form of an X-ray tube for a good tube, a bad tube, and a broken tube;
Fig. 8 shows a schematic example of a transceiver system in the form of a directional coupler;
Fig. 9 shows a schematic example of a transceiver system in the form of a coupler; and
Fig. 10 shows a schematic example of a transceiver system in the form of a coupler.

### DETAILED DESCRIPTION OF EMBODIMENTS

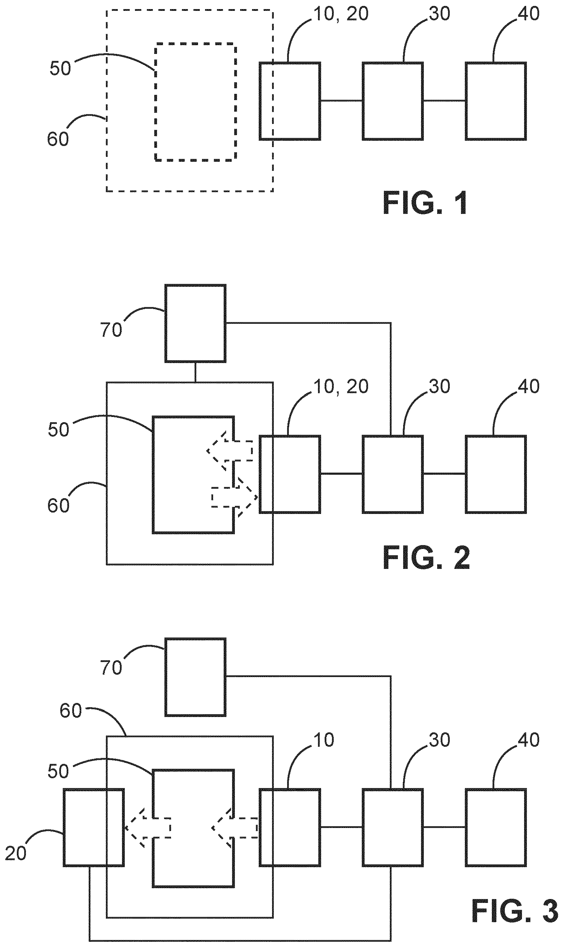

Fig. 1 shows an example of an X-ray imaging system failure detection apparatus. The apparatus comprises a transceiver system 10, 20, an analyser 30, and an output unit 40. The transceiver system is configured to be located relative to a component 50 of the X-ray imaging system 60. The transceiver system is configured to emit RF radiation. The transceiver system is configured to irradiate at least one part of the component with first RF radiation that is at least a portion of the emitted RF radiation. The transceiver system is configured to collect second RF radiation. The second RF radiation is at least a portion of the first RF radiation that is reflected and/or transmitted by the component. The transceiver system is configured to provide the analyser with data indicative of the second RF radiation. The analyser is configured to determine that the component has a fault or is developing a fault. The determination that the component has a fault or is developing a fault comprises utilization of the data indicative of the second RF radiation. The output unit is configured to output information that the component has a fault or is developing a fault upon the determination by the analyser that the component has a fault or is developing a fault.

The transceiver system can have a separate transmitter and receiver, or the transmitter and receiver can be housed in a single unit. Even when a transmitter and receiver are housed in the same unit, there can be some RF radiation that is transmitted as well as reflected, due to for example scattering within the component or reflection within the component, and where some RF radiation can be then reflected as well as transmitted back to the source of the radiation.

Thus, the way in which a component interacts with RF radiation can be used to detect a change in that interaction to assess or determine that the component has a fault or is developing a fault. The component can then be changed or repaired before problems occur, and the X-ray imaging system can be deactivated or have its mode of operation altered to mitigate the effect of the fault that has occurred or is developing in the component.

Thus, a new technique is provided to assess the functioning of a component using RF radiation, that would not be assessed with respect to RF radiation operation because it does not use RF radiation, but where the way in which it interacts with RF radiation intentionally irradiating it can be used to determine if the component has a fault.

It is noted that the RF radiation could be at a single frequency, or at a number of frequencies, or be quasi broadband and over a range of frequencies. The RF radiation can be from about 10kHz to a few GHz might be used.

Thus, the most information can be gained from "wide band" measurements, i.e. full spectra of transmitted or reflected RF signals (e.g. resonance frequencies, line shapes etc.). However, simpler measurements at selected frequencies can be sufficient to detect degradation and failures.

It is also noted that radiation reflected and/or transmitted by the component means radiation that has interacted with the component.

For example, the transceiver can be a single transceiver unit located at one location that emits and collects radiation, and where that radiation will interact with a component and return to the transceiver unit and some of that the radiation can be directly reflected at the surface and some of that radiation can entered the component and be transmitted before being emitted back toward the transceiver.

For example, the transceiver can be separate transmitter and receiver units that could be placed either side of the component, or on one side of the component next to each other, or generally on one side of the component but spaced from one another. Radiation received by the receiver can be transmitted or can again be a combination of reflected and transmitted radiation.

In other words, there can be more transmitter/receiver combinations and either the reflected or the transmitted (from one transmitter to another receiver) signal can be analyzed.

It is noted that the apparatus could be integrated or permanently connected to the component.

It is also noted that the apparatus could be connected to various connections such as inputs/outputs of the component for periodic inspection.

In an example, the component is a device that is not configured to generate RF signals such as an X-ray tube or generator.

In an example, the RF radiation emitted by the transceiver system has a frequency or range of frequencies up to 500MHz.

In an example, the RF radiation emitted by the transceiver system has a frequency or range of frequencies less than 100MHz and/or in the range 300-500MHz. It has been established that many components exhibit resonances in reflected RF radiation in these frequency ranges, and resonant frequencies are sensitive in terms of changes in the reflectance and/or transmittance of RF radiation at resonances shows enhanced changes when components have faults or are developing faults with respect to non-resonance frequencies.

According to an example, the transceiver system is configured to provide the analyser with data indicative of the first RF radiation. The determination that the component has a fault or is developing a fault can then comprise utilization of the data indicative of the first RF radiation to determine a normalized reflectance and/or transmittance.

Thus, in effect a form of normalised reflectance/transmittance can be determined. This can help analysis of data at different frequencies that can have different absolute magnitudes.

In an example, the emitted RF radiation is scanned over a frequency range, such as up to 100MHz or from 300-500MHz.

According to an example, the determination that the component has a fault or is developing a fault comprises a utilization of a change in the second RF radiation with respect to reference data.

Thus, the second RF radiation alone or a reflectance or transmittance when used with the first RF radiation can be used to determine a fault if the second RF has changed, where the second RF radiation or reflectance/transmittance can be compared for example with reference data for the component.

In an example, the change in the second RF radiation comprises a change in a magnitude and/or phase of the second RF radiation at one or more frequencies.

In an example, the one or more frequencies are resonant frequencies.

In an example, the change in the second RF radiation comprises a detection of a change in a frequency spectrum of the RF radiation.

According to an example, the RF radiation comprises radiation emitted over a period of time. The first RF radiation comprises radiation that irradiates the at least one part of the component over the period of time. The second RF radiation comprises radiation collected over the period of time. The determination that the component has a fault or is developing a fault can comprise a detection of a change in the second RF radiation over the period of time.

In this manner, by monitoring the interaction of RF radiation with the component, a change in that interaction can be used to determine that a fault has developed or is developing.

In an example, the period of time can be a second, a minute, an hour, a day, week, or month for example and the second RF radiation can be monitored periodically within this time to assess if there is a fault developing or if a fault has developed.

In an example, the period of time can be a second, a minute, an hour, a day, week, or month for example and the second RF radiation can be continually monitored, which enables an instantaneous change in the RF radiation help diagnose the fault with respect to the temporal variation in magnitude and/or phase. Thus, not only can it be determined that a fault is developing of has developed, but the type of fault can be identified.

According to an example, the detection of the change in the second RF radiation over the period of time comprises a detection of a change in a magnitude and/or phase of the second RF radiation at one or more frequencies.

According to an example, the one or more frequencies are resonant frequencies.

It has been established, that the interaction of RF radiation with components of an X-ray imaging system exhibits one or more resonances, in terms of for example minima in collected intensities of radiation at particular frequencies, and it has been established that as a fault develops in the component the change in intensity of collected radiation at these resonant frequencies is particularly pronounced. Thus, by monitoring these one or more resonant frequencies faults can be detected more effectively.

According to an example, the detection of the change in the second RF radiation over the period of time comprises a detection of a change in a frequency spectrum of the RF radiation.

Thus spectral analysis can be utilized, using standard spectral analysis tools, to detect if the spectrum is changing and therefore that a fault is developing or has developed.

According to an example, the determination that the component has a fault or is developing a fault comprises utilization of data indicative of one or more reference second RF radiation reflected and/or transmitted from the component or a reference component as a consequence of one or more reference first RF radiation having irradiated at least a part of the component or reference component.

Thus, for example for an apparatus integrated or permanently connected to a component, a comparison with reference data can be used to determine if there is a fault, and indeed identify a fault. However, the apparatus could be used to interrogate a component by connecting it to different connections of the component such as inputs and outputs to assess if there is a fault.

According to an example, the determination that the component has a fault or is developing a fault comprises utilization of data indicative of the one or more reference first RF radiation.

In other words, reference RF data that for example was acquired for components that are known not to have faults can have been previously determined and then the newly acquired second RF radiation and/or reflectance or transmittance data can be compared with this data to determine is there is a fault or a fault developing.

According to an example, the determination that the component has a fault or is developing a fault comprises an identification of the fault or identification of the fault that is developing.

In an example, the identification of the fault comprises utilization of the data indicative of the one or more reference second RF radiation reflected and/or transmitted from the component or the reference component.

In an example, the identification of the fault comprises utilization of the data indicative of the one or more reference first RF radiation

Thus, it can be determined that there is a fault when the second RF radiation has changed over time and/or is different to second RF radiation stored as reference data for a healthy component. However, available data for components that do have faults can also be utilized in order that a fault can be identified.

According to an example, the analyser is configured to determine that the component has a fault or is developing a fault during operation of the X-ray imaging system; and/or wherein the fault comprises at least one of: arcing in an X-ray tube of the X-ray imaging system, arcing in a generator of the X-ray imaging system, degradation of an anode bearing of an X-ray tube of the X-ray imaging system, and degradation of a filament of an X-ray tube of the X-ray imaging system.

Thus, the apparatus can determine that a component of the X-ray imaging system is faulty or is becoming faulty whilst the X-ray system is in operation, for example is radiating. The X-ray system is not based on RF technology, in contrast to some system that utilize RF radiating coils for example, and therefore the second RF radiation that is reflected and/or transmitted from the component of the X-ray system can be used to assess the health of components of the X-ray imaging system, because the imaging of the X-ray system can be separated from the RF based component health diagnostic system that has been developed.

In an example, the transceiver system comprises a single transceiver unit.

In an example, the transceiver unit is configured to be located adjacent to the component, located in contact with the component and/or located at least partially within the component.

In an example, the transceiver system comprises a transmitter unit 10 and a separate receiver unit 20.

In an example, the transmitter unit and receiver unit are configured to be located at different positions relative to the component.

In an example, the component is one of an X-ray tube, a high voltage generator, a high voltage cable, high voltage connector, high voltage transformer,

In an example, the transceiver system comprises one or more of a directional coupler, a loop or coil, a magnetic dipole, an electric dipole, a galvanic connector, a high voltage capacitor, a high voltage switch, a toroid, and antenna.

Figs. 2-3 show examples of a medical imaging system. The system comprises an X-ray imaging system 60, a processing unit 70, a transceiver system 10, 20, an analyser 30, and an output unit 40. The processing unit is configured to control at least one mode of operation of the X-ray imaging system. The transceiver system is located relative to a component 50 of the X-ray imaging system. The transceiver system is configured to emit RF radiation. The transceiver system is configured to irradiate at least one part of the component with first RF radiation that is at least a portion of the emitted RF radiation. The transceiver system is configured to collect second RF radiation. The second RF radiation is at least a portion of the first HR RF radiation that is reflected and/or transmitted by the component. The transceiver system is configured to provide the analyser with data indicative of the second RF radiation. The analyser is configured to determine that the component has a fault or is developing a fault. The determination that the component has a fault or is developing a fault comprises utilization of the data indicative of the second RF radiation. The output unit is configured to output information that the component has a fault or is developing a fault upon the determination by the analyser that the component has a fault or is developing a fault.

In Fig. 2 the transceiver system 10, 20 has a single transceiver/receiver system, thus the transmitter and receiver are housed together.

In Fig. 3 the transceiver system 10, 20 is made up of a separate transmitter 10 and a separate receiver 20.

In an example, the component is a device that is not configured to generate RF signals such as an X-ray tube or generator.

In an example, the RF radiation emitted by the transceiver system has a frequency or range of frequencies up to 500MHz.

In an example, the RF radiation emitted by the transceiver system has a frequency or range of frequencies less than 100MHz and/or in the range 300-500MHz.

According to an example, the processing unit is configured to stop or modify one or more of the at least one mode of operation of the X-ray imaging system comprising utilization of the information that the component has a fault or is developing a fault.

Thus, if it is detected that a component of the X-ray imaging system is developing a fault or has developed a fault, the X-ray imaging system can be in effect stopped or have its mode of operation changed such that the component having a failure or developing a failure will not compromise the operation of the X-ray imaging system.

In an example, the transceiver system is configured to provide the analyser with data indicative of the first RF radiation. The determination that the component has a fault or is developing a fault can comprise utilization of the data indicative of the first RF radiation to determine a normalized reflectance and/or transmittance.

In an example, the emitted RF radiation is scanned over a frequency range, such as up to 100MHz or from 300-500MHz.

In an example, the determination that the component has a fault or is developing a fault comprises a utilization of a change in the second RF radiation with respect to reference data.

In an example, the change in the second RF radiation comprises a change in a magnitude and/or phase of the second RF radiation at one or more frequencies.

In an example, the one or more frequencies are resonant frequencies.

In an example, the change in the second RF radiation comprises a detection of a change in a frequency spectrum of the RF radiation.

In an example, the RF radiation comprises radiation emitted over a period of time, wherein the first RF radiation comprises radiation that irradiates the at least one part of the component over the period of time. The second RF radiation comprises radiation collected over the period of time. The determination that the component has a fault or is developing a fault can comprise a detection of a change in the second RF radiation over the period of time.

In an example, the period of time can be a second, a minute, an hour, a day, week, or month for example and the second RF radiation can be monitored periodically within this time to assess if there is a fault developing or if a fault has developed.

In an example, the period of time can be a second, a minute, an hour, a day, week, or month for example and the second RF radiation can be continually monitored, which enables an instantaneous change in the RF radiation help diagnose the fault with respect to the temporal variation in magnitude and/or phase. Thus, not only can it be determined that a fault is developing of has developed, but the type of fault can be identified.

In an example, the detection of the change in the second RF radiation over the period of time comprises a detection of a change in a magnitude and/or phase of the second RF radiation at one or more frequencies.

In an example, the one or more frequencies are resonant frequencies.

In an example, the detection of the change in the second RF radiation over the period of time comprises a detection of a change in a frequency spectrum of the RF radiation.

In an example, the determination that the component has a fault or is developing a fault comprises utilization of data indicative of one or more reference second RF radiation reflected and/or transmitted from the component or a reference component as a consequence of one or more reference first RF radiation having irradiated at least a part of the component or reference component.

In an example, the determination that the component has a fault or is developing a fault comprises utilization of data indicative of the one or more reference first RF radiation.

In an example, the determination that the component has a fault or is developing a fault comprises an identification of the fault or identification of the fault that is developing.

In an example, the identification of the fault comprises utilization of the data indicative of the one or more reference second RF radiation reflected and/or transmitted from the component or the reference component.

In an example, the identification of the fault comprises utilization of the data indicative of the one or more reference first RF radiation

In an example, the analyser is configured to determine that the component has a fault or is developing a fault during operation of the X-ray imaging system; and/or wherein the fault comprises at least one of: arcing in an X-ray tube of the X-ray imaging system, arcing in a generator of the X-ray imaging system, degradation of an anode bearing of an X-ray tube of the X-ray imaging system, and degradation of a filament of an X-ray tube of the X-ray imaging system.

In an example, the transceiver system comprises a single transceiver unit.

In an example, the transceiver unit is located adjacent to the component, located in contact with the component and/or located at least partially within the component.

In an example, the transceiver system comprises a transmitter unit and a separate receiver unit.

In an example, the transmitter unit and receiver unit are located at different positions relative to the component.

It is noted that when the transmitter unit and receiver unit are located at separate locations, the receiver unit can actually be more than one receiver unit that receiver the RF radiation simultaneously.

In an example, the component is one of an X-ray tube, a high voltage generator, a high voltage cable, high voltage connector, high voltage transformer,

In an example, the transceiver system comprises one or more of a directional coupler, a loop or coil, a magnetic dipole, an electric dipole, a galvanic connector, a high voltage capacitor, a high voltage switch, a toroid, and antenna.

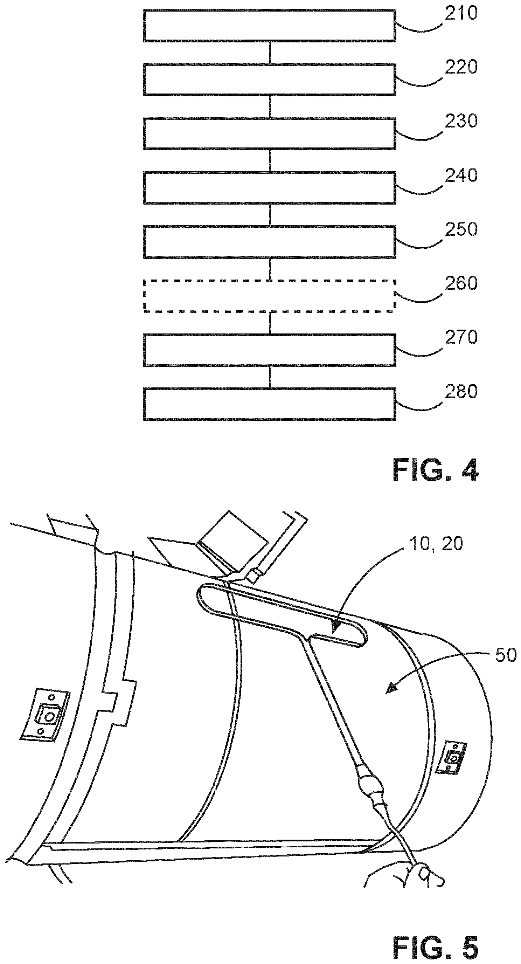

Fig. 4 shows an example of an X-ray imaging system failure detection method in its steps. The method comprises:
locating 210 a transceiver system relative to a component of the X-ray imaging system;
emitting 220 by the transceiver system RF radiation;
irradiating 230 by the transceiver system at least one part of the component with first RF radiation that is at least a portion of the emitted RF radiation;
collecting 240 by the transceiver system second RF radiation, wherein the second RF radiation is at least a portion of the first HR RF radiation that is reflected and/or transmitted by the component;
providing 250 by the transceiver system an analyser with data indicative of the second RF radiation;
determining 270 by the analyser that the component has a fault or is developing a fault, wherein the determining that the component has a fault or is developing a fault comprises utilizing the data indicative of the second RF radiation; and
outputting 280 by an output unit information that the component has a fault or is developing a fault upon the determination by the analyser that the component has a fault or is developing a fault.

In an example, the component is a device that is not configured to generate RF signals such as an X-ray tube or generator.

In an example, the RF radiation emitted by the transceiver system has a frequency or range of frequencies up to 500MHz.

In an example, the RF radiation emitted by the transceiver system has a frequency or range of frequencies less than 100MHz and/or in the range 300-500MHz.

In an example, the method comprises providing 260 by the transceiver system the analyser with data indicative of the first RF radiation, and the determining that the component has a fault or is developing a fault comprises utilizing the data indicative of the first RF radiation to determine a normalized reflectance and/or transmittance.

In an example, the method comprises scanning the emitted RF radiation over a frequency range, such as up to 100MHz or from 300-500MHz.

In an example, the determining that the component has a fault or is developing a fault comprises utilizing a change in the second RF radiation with respect to reference data.

In an example, the change in the second RF radiation comprises a change in a magnitude and/or phase of the second RF radiation at one or more frequencies.

In an example, the one or more frequencies are resonant frequencies.

In an example, the change in the second RF radiation comprises detecting a change in a frequency spectrum of the RF radiation.

In an example, the RF radiation comprises radiation emitted over a period of time, wherein the first RF radiation comprises radiation that irradiates the at least one part of the component over the period of time, the second RF radiation comprises radiation collected over the period of time, and the determining that the component has a fault or is developing a fault comprises detecting a change in the second RF radiation over the period of time.

In an example, the period of time can be a second, a minute, an hour, a day, week, or month for example and the second RF radiation can be monitored periodically within this time to assess if there is a fault developing or if a fault has developed.

In an example, the period of time can be a second, a minute, an hour, a day, week, or month for example and the second RF radiation can be continually monitored, which enables an instantaneous change in the RF radiation help diagnose the fault with respect to the temporal variation in magnitude and/or phase. Thus, not only can it be determined that a fault is developing of has developed, but the type of fault can be identified.

In an example, the detecting the change in the second RF radiation over the period of time comprises detecting a change in a magnitude and/or phase of the second RF radiation at one or more frequencies.

In an example, the one or more frequencies are resonant frequencies.

In an example, the detecting the change in the second RF radiation over the period of time comprises detecting a change in a frequency spectrum of the RF radiation.

In an example, the determining that the component has a fault or is developing a fault comprises utilizing data indicative of one or more reference second RF radiation reflected and/or transmitted from the component or a reference component as a consequence of one or more reference first RF radiation having irradiated at least a part of the component or reference component.

In an example, the determining that the component has a fault or is developing a fault comprises utilizing data indicative of the one or more reference first RF radiation.

In an example, the determining that the component has a fault or is developing a fault comprises identifying the fault or identifying the fault that is developing.

In an example, the identifying the fault comprises utilizing the data indicative of the one or more reference second RF radiation reflected and/or transmitted from the component or the reference component.

In an example, the identifying the fault comprises utilizing the data indicative of the one or more reference first RF radiation.

In an example, the method comprises utilizing the analyser to determine that the component has a fault or is developing a fault during operation of the X-ray imaging system; and/or wherein the fault comprises at least one of: arcing in an X-ray tube of the X-ray imaging system, arcing in a generator of the X-ray imaging system, degradation of an anode bearing of an X-ray tube of the X-ray imaging system, and degradation of a filament of an X-ray tube of the X-ray imaging system.

In an example, the transceiver system comprises a single transceiver unit.

In an example, the transceiver unit is located adjacent to the component, located in contact with the component and/or located at least partially within the component.

In an example, the transceiver unit comprises a transmitter unit and a separate receiver unit.

In an example, the transmitter unit and receiver unit are located at different positions relative to the component.

In an example, the component is one of an X-ray tube, a high voltage generator, a high voltage cable, high voltage connector, high voltage transformer,

In an example, the transceiver system comprises one or more of a directional coupler, a loop or coil, a magnetic dipole, an electric dipole, a galvanic connector, a high voltage capacitor, a high voltage switch, a toroid, and antenna.

The X-ray imaging system failure detection apparatus, medical imaging system, and X-ray imaging system failure detection method are now described in further detail, where reference is made to Figs. 5-10.

The following relates to failure detection with respect to components of an X-ray imaging system. The described techniques also focus on arcing failures in such an X-ray imaging systems, but other failure modes for X-ray imaging systems, such as relating to failure of drives, power supplies, bearings etc can be detected.

As discussed above, arcing causes issues in medical X-ray and CT systems (generally termed X-ray imaging systems). It causes system downtime and the repair can be cumbersome, if the arcing location and thus the component to be fixed is not known. Since arcing is mostly related to a degrading or an imperfection of some component (bubbles in oil, isolation loss, soot on connectors, bad tube vacuum, loose contacts etc.) the inventors realized that there is an opportunity through appropriate sensing and analysis to predict the onset of arcing by monitoring the characteristics of system components. In this way downtime can be avoided and precise information can be provided as to the components involved. As detailed above, also other faults than arcing provoking conditions can in be predicted/determined by the new technique.

The new technique provides data for predicting/ detecting a failure of an X-ray imaging system by measuring the coupling of RF fields into components/structures (e.g. tube, generator) of the X-ray imaging system. The measurements represent the electromagnetic characteristics of these components. The corresponding measurements can in principle be performed without affecting the operation of the imaging unit. The high frequency electromagnetic properties and thus the response to high frequency RF (RF) excitation characterize a given structure - reference is made here to high frequency RF, however the new technique can operate at what the skilled person would consider to be normal radio frequencies and need not be HF. Thus, reference is generally made to RF rather than HF, however where reference to RF refers to a potentially broad spectrum of radio frequencies. Local changes in geometry or electromagnetic properties (conductivity, dielectric constant) are thus reflected in the response to an RF excitation, e.g. at the inherent resonance frequencies of the structure. The response to RF excitations is measured at different frequencies and changes of these responses over time or in comparison to reference measurements are used to monitor degradation and predict malfunction. In detail, it is thus proposed to install dedicated coupling structures onto, next to or into the components of the X-ray imaging system, which allow to couple RF signals into and out of X-ray imaging system components. Alternatively, already existing contacts of the X-ray imaging system can be used as coupling structures, to feed in and read out the RF response.

The inventors realized that an active technique, transmitting and receiving RF via a coupling structure (transceiver system) could characterize components and determine that a problem was developing prior to an arcing event (or other fault) actually fully occurring.

The new technique involves transmitting RF signals into the a component 50 of an X-ray imaging unit or system 60 at various frequencies and receiving the corresponding response using a transceiver system 10,20, in other words, measuring the transmitted and reflected waves at an analyzer 30. The transmitter and receiver may be provided by a so-called network analyzer.

A transceiver system 10, 20, that can be considered to be a coupling structure, is therefore located next to, mounted to or embedded within a component 50 of the X-ray imaging system 60 to provide measurements of the RF responses, in particular the resonances, that provides a good characterization of the components, This allows for detection of an upcoming malfunction and degradation. As an example, soot in the vicinity of RF current carrying structures will couple electrically and will lead to additional losses, so that the quality factor and the resonance frequency of a corresponding RF resonance will be changed, and this can be detected. Similarly, air bubbles in the insulating oil will change the effective dielectric constant leading to resonance frequency shifts, which can be detected.

Fig. 5 shows a technique of measuring the RF response of a structure 50 by transceiver system 10, 20 in the form of a pick-up coil used for example in the context of MRI RF coils.

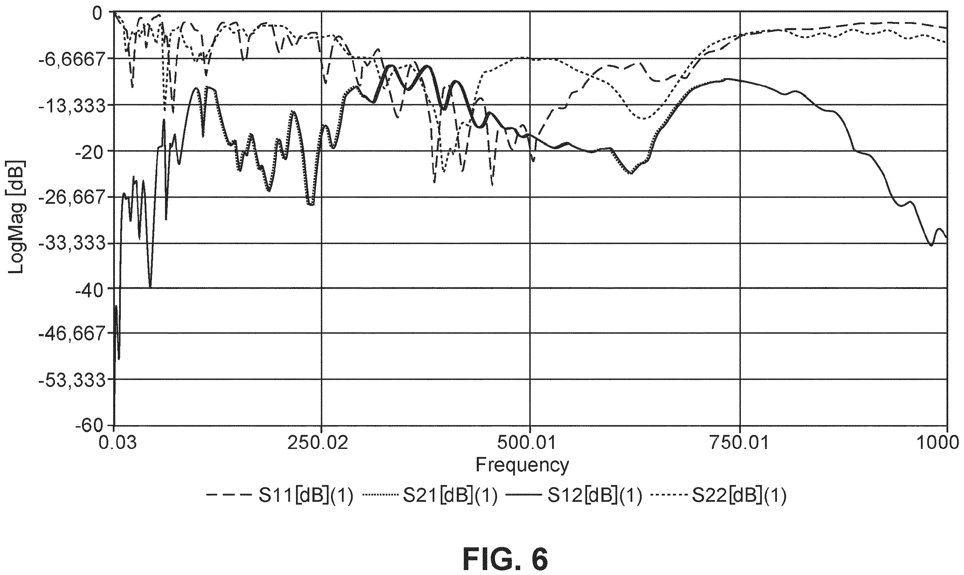

Fig. 6 shows RF reflection and transmission measurements versus frequency (MHz) performed on the anode and cathode of an X-ray tube, where S11, S22, S12 and S21 are measured by a network analyzer at the anode and cathode of the X-ray tube. Several sharp "troughs" indicate the presence of resonances, e.g. below approximately 100 MHz and in the range of approximately 300MHz-500MHz. These measurements were performed by direct galvanic connection of a network analyzer to a single tube.

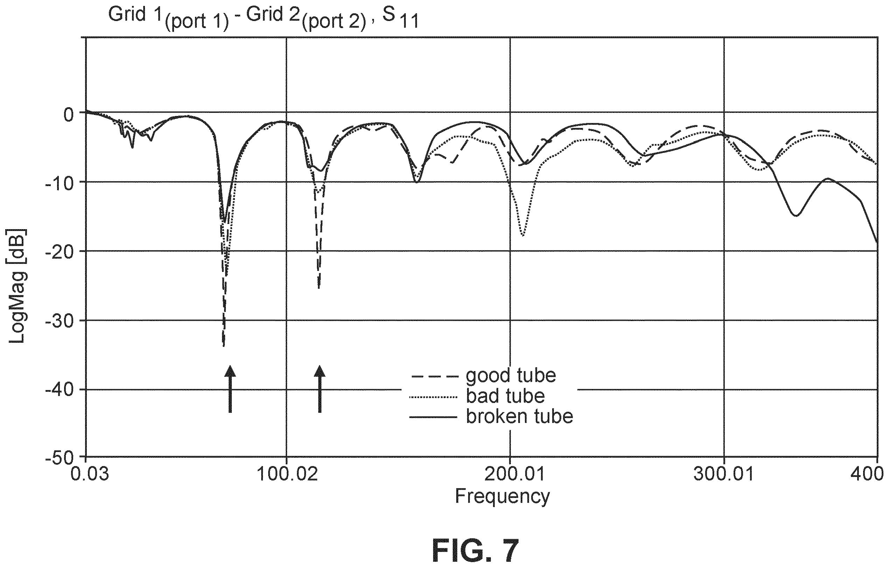

Fig. 7 shows reflection measurements versus frequency (MHz) for S 11 for an X-ray tube, that is a component 50 of an X-ray imaging system 60, for a good tube, a bad tube and a broken tube measured by an analyzer 30. In Fig. 7, the good tube is a normally-functioning tube, the bad tube is a tube in which the anode bearing has degraded, and the broken tube is a tube in which the anode bearing has completely failed and arcing has occurred. The good tube shows the deepest resonance troughs at the positions of the arrows, with the bad tube showing the next deepest troughs, and the broken tube shows the most shallow trough. A distinction between the bad tube (degraded anode bearing) and the broken tube (anode bearing failed, arcing) is also visible via a shift in the resonance trough in the frequency interval 300 - 400 MHz. Thus, the measurements can determine if an X-ray tube is operating correctly, and if not operating correctly the degree of failure can be determined. The type of fault can also be determined. This information facilitates a decision on a subsequent course of action, such as an automatic adjustment to the mode of operation of the component that facilitates continued operation of the component with reduced risk of complete failure, or an automatic shut-down of the component, or an automatic termination of the present imaging examination, or a continuation of the present imaging examination, or an automatic request for a serving operation. Such operations may be implemented via an output unit 40.

The coupling of RF signals into X-ray system components during normal operation is challenging, since high DC voltages are present and several components are (partially) shielded, however it can be achieved as the X-ray imaging system is not an RF active system as such, the RF signals can be analyzed without interference from the X-ray imaging system imaging modality signals.

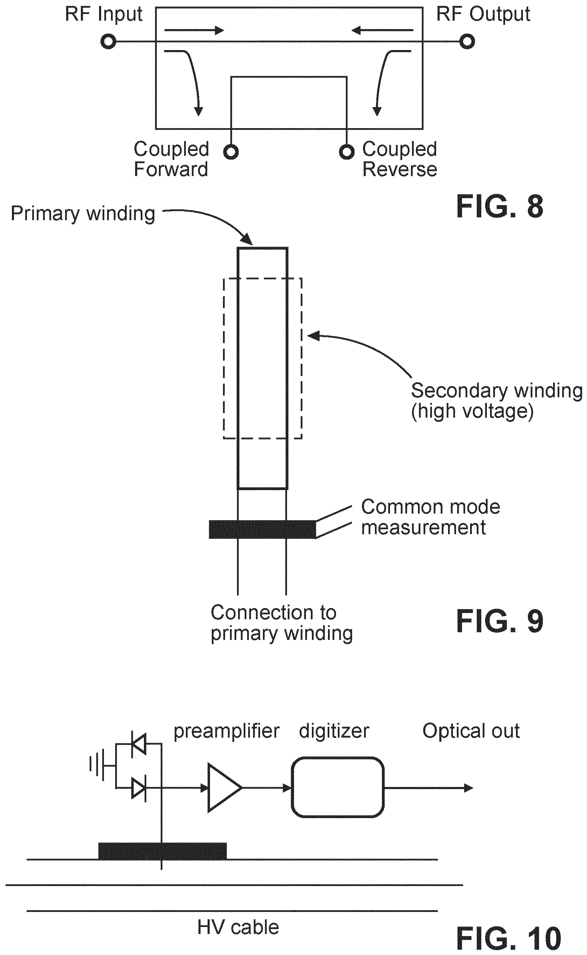

The inventors have derived a number of transceiver system 10, 20 means to enable the ability to perform analysis of components of an X-ray imaging system whilst it is operating:
Implementation of directional couplers e.g. in the HV cable, where Fig. 8 shows an example of a transceiver system 10, 20 in the form of a directional coupler. This can additionally provide information on signal direction. Since a HV coaxial cable is a type of RF cable, it can be possible to implement appropriate EM coupled ports even in the presence of the high DC voltages.

Coupling via loops (magnetic dipoles) or short electric dipoles, depending on the frequency and position.

Implementation of coupling loops/ dipoles at the HV connectors. At the connection interface there can be sufficient space, e.g. on the ceramics where flat coupling structures can be placed without getting too close to the high voltages. Moreover, at this position, there is still a slight gap in the shielding, respectively the loop can even be placed at the inside of the metal container.

Coupling at the transformer of the generator (e.g. by an additional coupling loop). At this position, the applied voltages are not as high as at the generator output.

Two loops can be placed on opposite sides of an oil tank and two-port measurements can be made. Based on the air or impurity content of the oil and the resulting change in dielectric properties, the differences should become visible in the spectrum.

The coupling structures (transceiver system 10, 20) and the corresponding Rx/Tx equipment including subsequent processing (analyzer 30) can also be configured as an upgrade kit for older systems. Alternatively, the RF coupling can be implemented by direct galvanic connections, either applying high voltage capacitors (if a sufficient voltage rating is available) or switches. In the latter case the measurements are however not independent from the operation of the X-ray generation. However, the RF measurements do not have to be performed during system operation, and can instead be scheduled to take place when the system is idle. This can also simplify using already existing contacts of the system as coupling structures, to feed in and read out the RF response.

In terms of data analysis, mainly the frequency response of the structure is observed (magnitude and phase). Either whole reflection spectra or tables with a number of selected (resonance) frequencies, their amplitudes and the Area under curves (AUCs) or intensity of spectrum (the peak area) can be recorded and analyzed. Special attention can be paid to resonances, since their position and quality factor can be determined, and these values are strongly related to the EM properties and the geometry of the device. Changes over time or in comparison to a reference measurement indicate degradation or possible malfunction. Correlating the measurements with arcing events or other defects provides more detailed knowledge on when and which action has to be taken. For cost reasons it is not essential to use a complete network analyzer, but the transmission of one or multiple defined spectra or individual wavelengths and the analysis of differences of the received spectrum or individual wavelengths over time allows for cost efficient hardware solutions. Measurements can be performed in dedicated analysis scans when also different defined operation conditions are used and can be reproduced for comparison measurements. In order not to have to introduce a dedicated system diagnosis scan, usage of for example the first scout scan of each day (or week) is conceivable. However, measurements can also be performed outside of normal system operation times when the system is idle. This can ensure reproducible conditions e.g. w r t temperature, which can have an influence on e.g. various distances and influence the reflection spectra, however the new technique can operate with working X-ray imaging units/systems that enables immediate remedial action to be taken when a component is seen to be failing, such as aborting a scan or exam or changing the exam or can slightly in order that the component is not so highly "stressed", and is likely to be able to be used until the end of the exam or scan, thereby obviating the need for the patient to receive a repeat scan or exam. Also, the positioning of cables for example may influence the spectral response, but can be taken into account with simple set-up tests and calibrations. It is noted that the analysis of certain resonance frequencies can be effective, however full spectrum analysis using for example partial least squares analysis or principle component analysis with respect to reference data can detect failure modes as components are starting to fail.

As discussed above, any correlation analysis technique, such as PLS, PCA or the use of neural networks, machine learning or AI-based analysis or simple single frequency (e.g. resonant frequency) monitoring can be used to detect the differences over time, and with data from comparable systems and defined failures, also a prediction of the type of failure, the location and the gradient of performance change could be processed. This data can lead to failure prediction and dedicated component stress profiles.

The new technique can be implemented in X-ray and high voltage devices like X-ray tubes, high voltage connectors, cables, high voltage generators, rotation systems, bearing systems, drive systems, where for example monitoring is carried out at dedicated locations of these devices that are known to be susceptible to failure. Electronics can be operated at defined voltage potential to avoid additional isolation problems and data transmission of the analysed and processed date can be transferred via different communication channels - e.g. optically.

Measurement of common mode and differential mode by non-galvanic measurement using sensor (toroids) can be utilized, where sensed data can be directly digitized in-place to prevent coupling to feeding lines if necessary. Coupling structures for sensitive electrical field measurement can be realized using an active antenna using a ground plane antenna, which is highly sensitive to spark incidents due to the high impedance at the antenna. A local preamplifier including digitalization can be placed on the HV cable using an electrical sensor with a dielectric fixation. The whole unit can be HV protected by the optical connection for digital signal transmission and power supply. This type of sensor can also be realized as a sniffing sensor to couple to critical or sensitive positions. Example transceiver systems 10, 20 are shown in Figs. 9-10 that show different coupling structures for RF measurements as predictive and failure sensors. Direct sensing of common mode currents on primary winding is shown in Fig. 9. Sensing of electrical field using short ground plane antenna and preamplifier with a protection unit is shown in Fig. 10.

As discussed above, instead of using wide frequency impedance analysis, narrow-band single-shot measurements at critical frequencies can be realized using low-cost transmitters using quartz or digital synthesis. Discrete frequencies can be measured simultaneously. Different sensors can be distributed on the X-Ray imaging system and a diversity unit combines the signals from different coupling structures that are mounted at separate locations. The coupling signal measuring resonance effects can be used to detect critical situations. The signal can be further fed to a control unit to change different parameters such as (IGBT switching frequency, voltage, phase, start/stop).

In another example embodiment, a computer program or computer program element is provided that is characterized by being configured to execute the method steps of any of the methods according to one of the preceding embodiments, on an appropriate apparatus or system.

The computer program element might therefore be stored on a computer unit, which might also be part of an embodiment. This computing unit may be configured to perform or induce performing of the steps of the method described above. Moreover, it may be configured to operate the components of the above-described system. The computing unit can be configured to operate automatically and/or to execute the orders of a user. A computer program may be loaded into a working memory of a data processor. The data processor may thus be equipped to carry out the method according to one of the preceding embodiments.

This example embodiment of the invention covers both, a computer program that right from the beginning uses the invention and computer program that by means of an update turns an existing program into a program that uses the invention.

Further on, the computer program element might be able to provide all necessary steps to fulfill the procedure of an example embodiment of the method as described above.

According to a further example embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further example embodiment of the present invention, a medium for making a computer program element available for downloading is provided, which computer program element is arranged to perform a method according to one of the previously described embodiments of the invention.

It has noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or examples and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting their scope.

## Claims

1. An X-ray imaging system failure detection apparatus, the apparatus comprising:
- a transceiver system (10, 20);
- an analyser (30); and
- an output unit (40);
wherein the transceiver system is configured to be located relative to a component (50) of the X-ray imaging system (60),
wherein the transceiver system is configured to emit RF radiation;
wherein the transceiver system is configured to irradiate at least one part of the component with first RF radiation that is at least a portion of the emitted RF radiation;
wherein the transceiver system is configured to collect second RF radiation, wherein the second RF radiation is at least a portion of the first RF radiation that is reflected and/or transmitted by the component;
wherein the transceiver system is configured to provide the analyser with data indicative of the second RF radiation;
wherein the analyser is configured to determine that the component has a fault or is developing a fault, wherein the determination that the component has a fault or is developing a fault comprises utilization of the data indicative of the second RF radiation; and
wherein the output unit is configured to output information that the component has a fault or is developing a fault upon the determination by the analyser that the component has a fault or is developing a fault.

2. The X-ray imaging system failure detection apparatus according to claim 1, wherein the transceiver system is configured to provide the analyser with data indicative of the first RF radiation, and wherein the determination that the component has a fault or is developing a fault comprises utilization of the data indicative of the first RF radiation to determine a normalized reflectance and/or transmittance.

3. The X-ray imaging system failure detection apparatus according to any of claims 1-2, wherein the determination that the component has a fault or is developing a fault comprises a utilization of a change in the second RF radiation with respect to reference data.

4. The X-ray imaging system failure detection apparatus according to any of claims 1-3, wherein the RF radiation comprises radiation emitted over a period of time, wherein the first RF radiation comprises radiation that irradiates the at least one part of the component over the period of time, wherein the second RF radiation comprises radiation collected over the period of time, and wherein the determination that the component has a fault or is developing a fault comprises a detection of a change in the second RF radiation over the period of time.

5. The X-ray imaging system failure detection apparatus according to claim 4, wherein the detection of the change in the second RF radiation over the period of time comprises a detection of a change in a magnitude and/or phase of the second RF radiation at one or more frequencies.

6. The X-ray imaging system failure detection apparatus according to claim 5, wherein the one or more frequencies are resonant frequencies.

7. The X-ray imaging system failure detection apparatus according to any of claims 4-6, wherein the detection of the change in the second RF radiation over the period of time comprises a detection of a change in a frequency spectrum of the RF radiation.

8. The X-ray imaging system failure detection apparatus according to any of claims 1-7, wherein the determination that the component has a fault or is developing a fault comprises utilization of data indicative of one or more reference second RF radiation reflected and/or transmitted from the component or a reference component as a consequence of one or more reference first RF radiation having irradiated at least a part of the component or reference component.

9. The X-ray imaging system failure detection apparatus according to claim 7, wherein the determination that the component has a fault or is developing a fault comprises utilization of data indicative of the one or more reference first RF radiation.

10. The X-ray imaging system failure detection apparatus according to any of claims 1-8, wherein the determination that the component has a fault or is developing a fault comprises an identification of the fault or identification of the fault that is developing.

11. The X-ray imaging system failure detection apparatus according to any of claims 1-10, wherein the analyser is configured to determine that the component has a fault or is developing a fault during operation of the X-ray imaging system; and/or wherein the fault comprises at least one of: arcing in an X-ray tube of the X-ray imaging system, arcing in a generator of the X-ray imaging system, degradation of an anode bearing of an X-ray tube of the X-ray imaging system, and degradation of a filament of an X-ray tube of the X-ray imaging system.

12. A medical imaging system comprising:
- an X-ray imaging system (60);
- a processing unit (70);
- a transceiver system (10, 20);
- an analyser (30); and
- an output unit (40);
wherein the processing unit is configured to control at least one mode of operation of the X-ray imaging system;
wherein the transceiver system is located relative to a component (50) of the X-ray imaging system,
wherein the transceiver system is configured to emit RF radiation;
wherein the transceiver system is configured to irradiate at least one part of the component with first RF radiation that is at least a portion of the emitted RF radiation;
wherein the transceiver system is configured to collect second RF radiation, wherein the second RF radiation is at least a portion of the first HR RF radiation that is reflected and/or transmitted by the component;
wherein the transceiver system is configured to provide the analyser with data indicative of the second RF radiation;
wherein the analyser is configured to determine that the component has a fault or is developing a fault, wherein the determination that the component has a fault or is developing a fault comprises utilization of the data indicative of the second RF radiation; and
wherein the output unit is configured to output information that the component has a fault or is developing a fault upon the determination by the analyser that the component has a fault or is developing a fault.

13. The medical imaging system according to claim 12, wherein the processing unit is configured to stop or modify one or more of the at least one mode of operation of the X-ray imaging system comprising utilization of the information that the component has a fault or is developing a fault.

14. An X-ray imaging system failure detection method, the method comprising:
locating (210) a transceiver system relative to a component of the X-ray imaging system;
emitting (220) by the transceiver system RF radiation;
irradiating (230) by the transceiver system at least one part of the component with first RF radiation that is at least a portion of the emitted RF radiation;
collecting (240) by the transceiver system second RF radiation, wherein the second RF radiation is at least a portion of the first HR RF radiation that is reflected and/or transmitted by the component;
providing (250) by the transceiver system an analyser with data indicative of the second RF radiation;
determining (270) by the analyser that the component has a fault or is developing a fault, wherein the determining that the component has a fault or is developing a fault comprises utilizing the data indicative of the second RF radiation; and
outputting (280) by an output unit information that the component has a fault or is developing a fault upon the determination by the analyser that the component has a fault or is developing a fault.

15. A computer program element for controlling an apparatus according to any of claims 1-11 which when executed by a processor is configured to carry out the method of claim 14, or for controlling a system according to any of claims 12-13 which when executed by a processor is configured to carry out the method of claim 14.
